# EUROPEAN PATENT APPLICATION

(11) **EP 2 335 744 A1**
(43) Date of publication of application: **22.06.2011**
(21) Application number: 09809951.8
(22) Date of filing: 26.08.2009
(51) Int. Cl.: A61L 27/00

(54) **BONE-FILLING TYPE AGENT FOR INDUCING CARTILAGE REGENERATION**

(30) Priority: 26.08.2008 JP 2008217253
(71) Applicant: National University Corporation Hokkaido University, Sapporo-shi Hokkaido 060-0808 (JP)
(72) Inventor: YASUDA, Kazunori, . (JP); OSADA, Yoshihito, . (JP); GONG, Jian Ping, . (JP); KITAMURA, Nobuto, . (JP)
(74) Representative: Prüfer & Partner GbR European Patent Attorneys
(86) International application number: PCT/JP2009/064888
(87) International publication number: WO 2010/024300

(57) **Abstract**

Provided is a medical material which enables a novel treatment method for cartilage regeneration being different from a treatment method through transplantation of autologous cartilage, a cartilage alternative or undifferentiated cells. Provided is a bone-filling type agent for inducing cartilage regeneration comprising a hydrogel which contains, as a monomer unit, an acid having an acidic group and/or a salt obtained by adding a metal to an acidic group and does not contain an interpenetrating network structure or a semi-interpenetrating network structure composed of two or more polymers. The bone-filling type agent for inducing cartilage regeneration can induce natural cartilage regeneration in the body by filling it in a hole or a groove provided in subchondral bone beneath cartilage defect.

## Description

### Technical Field

The present invention relates to a bone-filling type cartilage regeneration inducer useful in a regeneration therapy for the cartilage tissue in the joint tissue.

### Background Art

In the tissue of a joint such as the knee joint and the shoulder joint, the tips of the bones, which are connected by the joint, are each covered with the cartilage tissue such that the bone is not directly rubbed with the other bone. When the cartilage tissue is damaged by e.g., aging, application of excessive load or repeated application of load, inflammation occurs within the joint and will be experienced as so-called joint pain. The articular cartilage tissue damage and joint pain widely and frequently occur from young to older people. In view of improving QOL (Quality of Life) of the patients or healthcare economy, development of an effective and rational therapy has been desired.

In the therapy for the damaged cartilage tissue, the most important issue is that the cartilage tissue does not spontaneously regenerate in the living body and thus it is extremely difficult to regenerate the damaged cartilage tissue by drug administration, etc. For example, in a microfracture method, in which a large number of small holes are punched in the subchondral bone, fibrocartilage is formed; however, healthy articular cartilage, i.e., hyaline cartilage, does not regenerate. For this reason, damaged cartilage tissue is presently cured exclusively by implantation of the cartilage tissue taken from a living body.

The implantation therapies by the cartilage tissue taken from a living body are roughly divided into two groups, an autologous cartilage implantation therapy (mosaic plasty) and cultured autologous cartilage implantation therapy. The autologous cartilage implantation therapy (mosaic plasty) is a method in which the autologous cartilage tissue having a bone plug attached thereto is taken from the undamaged part of the damaged joint or the opposite articular tissue and auto-implanted in a defect. However, this therapy inevitably has a problem in that when cartilage is taken from a site, the healthy cartilage of the site is damaged, and a problem in that when the size of damaged cartilage site is large, a cartilage tissue sufficient to subject to implantation cannot be taken from a living body. On the other hand, the cultured autologous cartilage implantation therapy is a method in which part of a patient's own cartilage (hyaline cartilage) tissue is taken, cultured with an appropriate medium and/or a culture substratum to regenerate an in-vitro cartilage tissue, which is implanted in an affected site of the patient. The cultured autologous cartilage implantation therapy has problems: for aseptically culturing the cartilage tissue, extremely expensive equipment is prepared; two operations must be performed for taking a cartilage tissue and implanting it; therapy cost increases due to e.g., long-term hospitalization of a patient; contamination during culturing poses a risk for causing a zoonotic infection; and uncertain therapy effect.

Also to avoid such problems, it is important to develop a therapy for facilitating spontaneous cartilage regeneration within a patient's own body. At present, a method for locally administering a cytokine such as b-FGF and OP-1 together with a carrier to the joint including damaged cartilage tissue and a method for administering autologous mesenchyme system stem cells and ES cells to the joint including damaged cartilage tissue are also experimentally studied. However, advantages of these methods and drawbacks thereof such as side effects are major tasks to be studied in the future. The therapy of this kind has not yet been put into practical use.

The present inventors have established a new cartilage regeneration therapy, which is different from a therapy based on implantation of an autologous cartilage tissue, a cartilage alternative or undifferentiated cells, on the basis of a completely novel concept. They found that a hydrogel (hereinafter referred to as a DN gel (double network gel)), which has an interpenetrating network structure or a semi-interpenetrating network structure formed of at least two polymers having a crosslinked network structure, is available as a bone-filling type cartilage regeneration inducer in the regeneration therapy for the cartilage tissue. The finding was,disclosed in International Patent Application (PCT/JP2007/001320) and the web site (Non Patent Literature 1).

The DN gel is a gel material having an interpenetrating network structure or a semi-interpenetrating network structure and excellent in strength; however, the interpenetrating network structure or semi-interpenetrating network structure formed of at least two polymers must be formed. Therefore, the complicated preparation of a gel is pointed out as a problem.

### Citation List

### Non Patent Literature

Non Patent Literature 1: Hokkaido University, Future drug development-medical innovation point formation, Medical research, Functional regeneration course, Motor function regeneration medical field,
   http://www.lfsci.hokudai.ac.jp/innovahome/team/index5.htm 1

### Summary of Invention

### Technical Problem

The present invention provides a novel cartilage regeneration inducer that can be used in place of a bone filler composed of the DN gel for use in a cartilage regeneration therapy.

### Solution to Problem

The present inventors surprisingly found that not the DN gel but a hydrogel having, as a monomer unit, sulfonic acid or a sulfonate, which is available as a polymer constituting the DN gel, can induce cartilage regeneration when a bone is filled solely with the hydrogel. Based on the finding, the following invention was accomplished.

(1) A bone-filling type cartilage regeneration inducer composed of a hydrogel which contains, as a monomer unit, an acid having an acidic group and/or a salt obtained by adding a metal to an acidic group and which does not contain either an interpenetrating network structure or a semi-interpenetrating network structure formed of at least two polymers.

(2) The bone-filling type cartilage regeneration inducer according to (1), wherein the acidic group is a carboxyl group, a phosphoric acid group or a sulfonic acid group.

(3) The bone-filling type cartilage regeneration inducer according to (2), wherein the acidic group is a sulfonic acid group.

(4) The bone-filling type cartilage regeneration inducer according to (1), wherein the hydrogel is a hydrogel in which 2-acrylamido-2-methylpropanesulfonic acid (AMPS) is polymerized and/or a hydrogel in which a salt obtained by adding a metal to a sulfonic acid group of 2-acrylamido-2-methylpropanesulfonic acid (AMPS) is polymerized.

### Advantageous Effects of Invention

The bone-filling type cartilage regeneration inducer of the present invention can facilitate cartilage regeneration by filling a hole or groove provided in the subchondral bone immediately beneath a damaged cartilage tissue, in the same manner as the DN gel, and can provide an extremely effective therapy completely free from problems involved in an autologous implantation method and a cultured autologous cartilage implantation therapy, as previously pointed out. Particularly, in the gel used in the present invention, an interpenetrating network structure and a semi-interpenetrating network structure need not be formed unlike in a DN gel, and therefore, the gel of the present invention is superior to a DN gel in view of gel preparation and moldability when a bone is filled therewith.

### Brief Description of Drawings

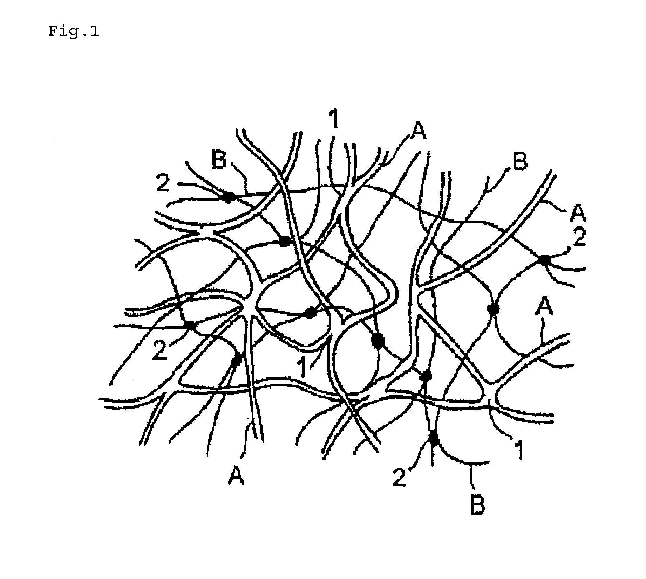

[Figure 1] Figure 1 is a schematic view of the "interpenetrating network structure" of the present invention, in which A represents a crosslinked network polymer; B represents another crosslinked network polymer, and 1 and 2 represent crosslinked points of respective crosslinked network polymers. Note that the figure is a conceptual view of a gel containing a solvent (water) in the network structure.

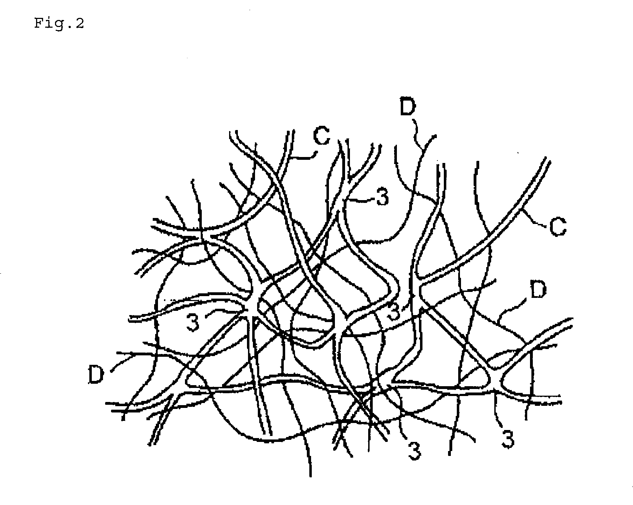

[Figure 2] Figure 2 is a schematic view of the "semi-interpenetrating network structure" of the present invention, in which C represents a crosslinked network polymer; D represents a linear polymer; and 3 represents a crosslinked point of the crosslinked network polymer.

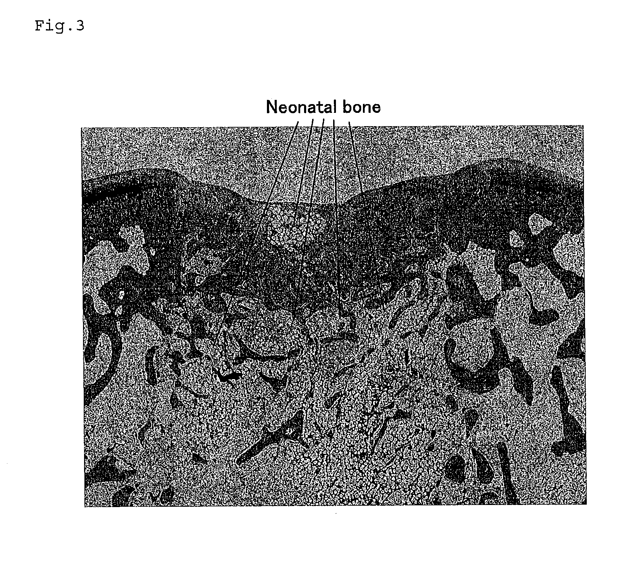

[Figure 3] Figure 3 is a photograph showing the Safranin O staining results of a bone defect in a non-treated control group (not filled with a PAMPS gel). In the photograph, the lower portion from the median depressed portion is a bone defect and the portion stained light green represents the cytoplasm and the portion remaining white represents the bone tissue.

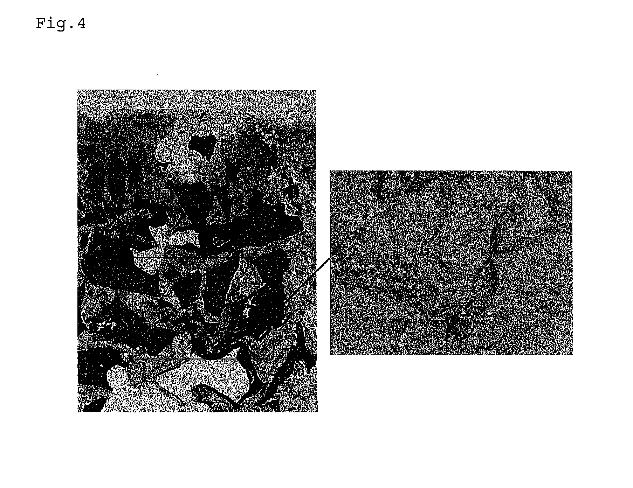

[Figure 4] Figure 4 shows photographs of Safranin O staining and type 2 collagen immunostaining results of a bone defect in a treated group (filled with a PAMPS gel). In the figure, the left one shows the Safranin O staining results; portion stained red represents a PAMPS gel; a portion stained pink or light-red represents a regenerated cartilage tissue; the portion stained light-green represents the cytoplasm; and a portion remaining white represents a bone tissue. The right figure is an enlarged view of a portion surrounded by the frame in the left figure, shows type 2 collagen immunostaining results of the same cut piece as used in the left figure; in which a portion stained brown represents a regenerated cartilage tissue.

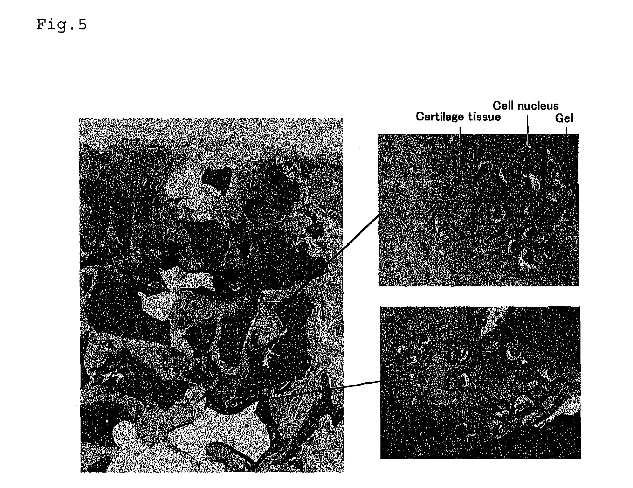

[Figure 5] Figure 5 shows the same photograph as the left figure of Figure 4 and an enlarged view of a portion surrounded by the frame. In the enlarged view, a portion stained dark represents a nucleus of cartilage cell; whereas a portion stained pink to light-red represents regenerated cartilage tissue.

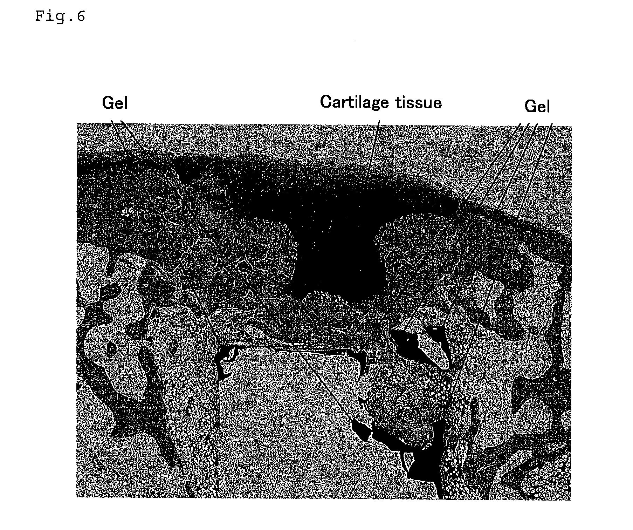

[Figure 6] Figure 6 is a photograph showing the Safranin O staining results of a bone defect in a treated group (filled with a PAMPS gel), which differs from the photographs of Figure 4 and Figure 5. In the figure, a portion stained red represents a PAMPS gel, a portion stained pink or light-red represents a regenerated cartilage tissue; a portion stained light-green represents the cytoplasm; and the portion stained white represents the bone tissue.

### Description of Embodiments

The present invention relates to a bone-filling type cartilage regeneration inducer composed of a hydrogel which contains, as a monomer unit, an acid having an acidic group and/or a salt obtained by adding a metal to an acidic group and which does not contain either an interpenetrating network structure or a semi-interpenetrating network structure formed of at least two polymers.

The acidic group in the present invention refers to a negatively charged group such as a carboxyl group, a phosphoric acid group, a sulfonic acid group and a sulfinic acid group. The acid having an acidic group means that a proton serves as a counter ion of the acidic group. Furthermore, the acidic group to which a metal is added, which results in a salt form by the addition of a metal, refers to the aforementioned negatively charged group. The salt obtained by adding a metal to an acidic group means that a positive metal ion serves as a counter ion of the acidic group. Hereinafter, if not otherwise specified, the acidic group and the acidic group to which a metal or a base is to be added will be collectively referred to as an "acidic group, etc.", and the acid having an acidic group and a salt obtained by adding a metal to the acidic group will be collectively referred to as an "electrolyte having the acidic group, etc.". Particularly preferable acidic group is a sulfonic acid group.

The hydrogel containing an "electrolyte having the acidic group, etc." as mentioned above as a monomer unit can be formed as a polymer, by polymerizing appropriate monomers having an "acidic group, etc." and a polymerizable chemical bond such as an unsaturated bond. Such a monomer, 2-acrylamido-2-methylpropanesulfonic acid (AMPS), acrylic acid (AA), methacrylic acid, styrenesulfonic acid or salts thereof such as sodium 2-acrylamido-2-methylpropane sulfonate, sodium acrylate and sodium styrenesulfonate may be mentioned.

Preferable examples of the hydrogel may include polyacrylamido-2-methylpropanesulfonic acid (PAMPS), which is obtained by polymerizing 2-acrylamido-2-methylpropanesulfonic acid (AMPS) as a monomer, and sodium poly(acrylamido-2-methylpropanesulfonate) (PNaAMPS) obtained by polymerizing sodium 2-acrylamido-2-methylpropanesulfate (NaAMPS) as a monomer.

Furthermore, the hydrogel having a salt obtained by adding a metal to an acidic group as a monomer unit may be prepared by obtaining a polymer by polymerizing appropriate monomers having an acidic group and a polymerizable chemical bond with each other and thereafter adding a metal to the acidic group in the polymer. For example, in physiological saline, it is conceivable that whole or part of counter ions (protons) is substituted by sodium ions to form a salt.

Note that, the hydrogel having an "electrolyte having an acidic group, etc." as a monomer unit may be a hydrogel having, as a monomer unit, only one of an acid having an acidic group and a salt obtained by adding a metal to the acidic group, or may be a hydrogel having, as monomer units, an acid having an acidic group and a salt obtained by adding a metal to the acidic group. Accordingly, the hydrogel having an "electrolyte having an acidic group, etc." as a monomer unit may be a homopolymer obtained by polymerizing a single monomer having either an acidic group or an acidic group to which a metal is added, or a copolymer obtained by polymerizing at least two monomers having an "acidic group, etc.". Furthermore, a copolymer obtained by copolymerizing a monomer having an "acidic group, etc." and a neutral group. In the latter case, the fraction of the monomer unit having the "acidic group, etc." is desirably 10% or more.

The aforementioned hydrogel is a hydrogel containing neither an interpenetrating network structure nor a semi-interpenetrating network structure. The interpenetrating network structure refers to a structure or state, for example, as shown in International Patent Application No. W02006/013612 [Figure 1] (cited as Figure 1 in this specification), in which at least two crosslinked network polymers are entangled so as to mutually go into network structures, with the result that a plurality of network structures are formed inside. The crosslinked network polymer herein refers to a polymer forming a network structure due to the presence of a plurality of crosslinking points. Furthermore the "semi-interpenetrating network structure" refers to a structure or state, for example, as shown in International Patent Application No. WO2006/013612, [Figure 2] (cited as Figure 2 in this specification), in which a linear polymer is entangled with a crosslinked network polymer so as to go into the mesh of the crosslinked network polymer, with the result that a plurality of network structures are formed inside.

The hydrogel is distinguished from the hydrogel described in Non Patent Literature 1 above in the respect that neither the interpenetrating network structure nor semi-interpenetrating network structure is contained. However, this means that the hydrogel of the present invention does not contain a network structure formed by complexly entangling at least two polymers and does not mean that the hydrogel of the present invention is limited to a hydrogel only consisting of a single polymer. More specifically, the hydrogel of the present invention may be not only a hydrogel consisting of a single polymer alone but also a mixture of hydrogels containing at least two polymers. As long as an interpenetrating network structure and a semi-interpenetrating network structure are not formed, polymers may be crosslinked.

Next, a method of using the aforementioned hydrogel as bone-filling type cartilage regeneration inducer will be described.

The hydrogel is used in a surgical operation for cartilage regeneration therapy. In the surgical operation, a hole or groove having an appropriate depth (hereinafter referred to as a bone defect) is formed by scraping the subchondral bone immediately beneath a damaged cartilage tissue together with the cartilage tissue, and the hole or groove is filled with the hydrogel. Note that, since most of hydrogels used in the invention of the present application are fragile, the hydrogel is sometimes used by finely crushing it, when a bone defect is filled with it. Such usage is also included in the present invention.

Artificial cartilage, which is a conventional therapeutic idea, is used not for expecting regeneration of cartilage but used as an alternative artificial material with which a damaged cartilage portion is to be filled so as to compensate for the damage. However, usage of the hydrogel completely differs from this. In the cartilage regeneration therapy by a surgical operation using the aforementioned hydrogel, neither implantation of the cartilage tissue taken from a living body nor administration of a special humoral factor facilitating cartilage regeneration is not required, spontaneous regeneration of a healthy cartilage tissue formed of hyaline cartilage is facilitated by providing a biological and mechanical environment for spontaneous cartilage regeneration by filling a bone defect with the hydrogel.

Furthermore, the hydrogel is atoxic to a living body when a bone is filled therewith. Thus, it is not necessarily required to remove the hydrogel from a bone defect after cartilage tissue is regenerated. Therefore, a patient who undergoes a surgical operation using the hydrogel just undergoes a single operation for providing a bone defect and filling the defect with the hydrogel. Therefore, the patient's burden is relieved compared to a conventional therapy requiring a plurality of operations.

As described above, the hydrogel is used for inducing cartilage regeneration by filling a bone defect provided in a damaged cartilage tissue with the hydrogel, and thus interpreted as an agent for inducing cartilage regeneration by filling the bone therewith. Therefore, the "bone-filling type" used in the present invention is interpreted as a term describing an instruction for use of the hydrogel in a surgical operation, that is, use of the hydrogel by filling a bone defect therewith. Furthermore, the "cartilage induction" is interpreted as a term describing an intended use or effect of the hydrogel, which is expected or exerted when the hydrogel is used as mentioned above, more specifically a term describing inducing or facilitating regeneration of cartilage tissue remaining around a bone defect.

Likewise, the present invention is directed to a bone-filling type cartilage regeneration inducer composed of the hydrogel and characterized by a "bone filling type" and a "cartilage regeneration inducer". In this respect, use of the present invention differs from that of artificial cartilage containing the hydrogel as a component polymer. Furthermore, although the hydrogel with which a bone defect has been filled is not directly in contact with either the cartilage tissue remaining around the bone defect or cartilage cells, the hydrogel has an effect of facilitating regeneration of cartilage cells or cartilage tissue. This suggests that the hydrogel does not serve as a direct "scaffold" for proliferating cartilage cells.

The present invention will be further specifically described by way of Production Examples and Usage Examples of the bone-filling type cartilage regeneration inducer of the present invention. However, these Examples are not construed as limiting the present invention.

### Examples

### <Example 1>

### (1) Preparation of hydrogel

A silicon board frame having a size of 80 mm long x 80 mm wide x 5 mm thick and a width of 5 mm was prepared. A groove of 3 mm was formed in a side of the board frame inwardly from the outside of the frame. The silicone board frame was sandwiched by two glass plates having a size of 100 mm long x 100 mm wide x 3 mm thick to assemble a polymerization container.

An aqueous solution (25 mL) of 2 mol/L 2-acrylamido-2-methylpropanesulfonic acid (AMPS), an aqueous solution (1 mL) of 2 mol/L N,N'-methylenebisacrylamide (MBAA) serving as a crosslinking agent, an aqueous solution (0.5 mL) of 0.1 mol/L 2-oxoglutaric acid and water were blended to prepare an aqueous solution (50 mL). The aqueous solution was deoxidized by use of nitrogen gas and poured through a groove of the polymerization container. After the groove was sealed, polymerization was performed by irradiation with ultraviolet light using a UV lamp (22 W, 0.34 A) of a wavelength of 365 nm at normal temperature for 6 hours to prepare a PAMPS gel sheet (thickness: 10 mm) having a degree of cross-linking of 4 mol%.

### (2) Surgical operation using hydrogel

Twenty mature Japanese white rabbits (3.0 to 4.2 kg) were prepared. A bone defect of 4.3 mm diameter and about 8 to 10 mm depth was formed on the thighbone side of the right knee patellofemoral joint (trochlea portion) of each rabbit. A PAMPS gel corresponding to the amount of rod (4.5 mm diameter) was taken from the PAMPS gel sheet prepared in the section (1), and the bone defect was filled with the gel so as to plug the defect. These were defined as a treated group. At this time, the bone defect was filled with the PAMPS gel such that the distance from the joint surface of the bone defect to the PAMPS gel surface was adjusted to maintain 2 mm.

Furthermore, a bone defect (diameter: 4.3 mm, depth: about 8 to 10 mm) was vertically formed on the thighbone side of the left knee thighbone/kneecap of each of the same Japanese white rabbits. These bone defects were not filled with any materials and defined as a non-treated control group.

Four weeks after the operation, the rabbits were raised in a cage and then killed. After the knee joint was observed with the naked eye, cut pieces were prepared from each of the right and left knee joints in a sagittal plane through the center of the damaged site and histological observation was made as described in 1) and 2) below.

### 1) Safranin O staining

Safranin O staining was performed in accordance with the method of Hirotani et al. (Clinical Orthopedic Surgery, Vol. 11, No. 12, pages 40-44, 1976). Furthermore, as counter staining, iron hematoxylin and fast green were used. By the staining, the PAMPS gel is indicated by red, the cartilage tissue is indicated by pink, the cell nucleus is indicated by dark purple, the cytoplasm is indicated by light green and the bone tissue is indicated by white.

### 2) Immunostaining of type 2 collagen

Type 2 collagen was immuno-stained in accordance with the method of Kumagai et al. (Kumagai et al., J. Anat., Vol. 185, pages 279-284, 1994). To describe more specifically, cut pieces were deparaffinized, dewatered, washed with water and then treated with Proteinase K at room temperature for 6 minutes. Thereafter, the cut pieces were washed with PBS, soaked in 1% hydrogen peroxide in methanol for 30 minutes, washed with PBS and incubated with a primary antibody diluted 50 fold (mouse anti-human collagen II antibody, Fuji Chemicals Industrial Co., Ltd.) at room temperature for 60 minutes. The cut pieces were washed again with PBS and incubated with a secondary antibody (anti-mouse IgG antibody, Envision) at room temperature for 30 minutes to immuno-stain type 2 collagen. By the staining, the PAMPS gel is stained purple and the cartilage tissue is stained brown.

The results of sections 1) and 2) show that the bone defects in the non-treated control group were filled with neonatal bone (Figure 3) but the cartilage tissue was not observed at all. On the other hand, in the right knees of rabbits having the bone defects filled with the PAMPS gel, it was confirmed that tissue was present so as to plug the gaps of the PAMPS gel with which the bone defect was filled, and stained with both Safranin O and type 2 collagen immunostaining (Figure 4, Figure 5 and Figure 6). The results of these two types of staining demonstrate that the tissue is a regenerated cartilage tissue.

Furthermore, it was confirmed by Safranin O staining that the cartilage tissue was regenerated so as not only to plug the gaps of the PAMPS gel with which the bone defect was filled but also to swell toward the surface of the open hole of the bone defect and in a broad area (Figure 6).

Note that, based on the aforementioned results, scores were given in accordance with the method of Wayne et al. (Wayne et al., Tissue Eng. Vol. 11, pages 953-963, 2005). Total average scores of the treated group and non-treated control group were 21.6 and 15.3, respectively. In a t-test, the p value was 0.0135. A significant difference between them is recognized.

## Claims

1. A bone-filling type cartilage regeneration inducer comprising a hydrogel which contains, as a monomer unit, an acid having an acidic group and/or a salt obtained by adding a metal to an acidic group and which does not contain either an interpenetrating network structure or a semi-interpenetrating network structure formed of at least two polymers.

2. The bone-filling type cartilage regeneration inducer according to claim 1, wherein the acidic group is a carboxyl group, a phosphoric acid group or a sulfonic acid group.

3. The bone-filling type cartilage regeneration inducer according to claim 2, wherein the acidic group is a sulfonic acid group.

4. The bone-filling type cartilage regeneration inducer according to claim 1, wherein the hydrogel is a hydrogel in which 2-acrylamido-2-methylpropanesulfonic acid (AMPS) is polymerized and/or a hydrogel in which a salt obtained by adding a metal to a sulfonic acid group of 2-acrylamido-2-methylpropanesulfonic acid (AMPS) is polymerized.
